(19) Europäisches Patentamt
European Patent Office
Office européen des brevets

(11) **EP 1 101 440 B2**

(12) **NEW EUROPEAN PATENT SPECIFICATION**
After opposition procedure

(45) Date of publication and mention
of the opposition decision:
**18.11.2009   Bulletin 2009/47**

(51) Int Cl.:
***A61B 5/021*** *(2006.01)*

(45) Mention of the grant of the patent:
**11.01.2006   Bulletin 2006/02**

(21) Application number: **99122743.0**

(22) Date of filing: **16.11.1999**

(54) **Non invasive blood pressure monitor**

Nichtinvasiver Blutdruckwächter

Appareil non invasif de surveillance de la pression sanguine

(84) Designated Contracting States:
**DE ES FR GB IT**

(43) Date of publication of application:
**23.05.2001   Bulletin 2001/21**

(73) Proprietor: **Microlife Intellectual Property GmbH
9443 Widnau (CH)**

(72) Inventors:
• **Forstner, Klaus
71679 Asperg/Württemberg (DE)**
• **Frick, Gerhard
6800 Feldkirch (AT)**
• **Yen, Chung-Yueh
Taichung 404 (TW)**

(74) Representative: **Hepp, Dieter et al
Hepp, Wenger & Ryffel AG,
Friedtalweg 5
9500 Wil (CH)**

(56) References cited:
**EP-A1- 0 342 249      EP-A1- 0 536 782
JP- - 04 227 227       JP-A- 7 227 743
US-A- 4 667 680        US-A- 5 649 536
US-A- 5 752 919**

• **NIPPON COLIN: 'User Manual', 01 June 1995
article 'Blood Pressure Monitor BP-203i'**

## Description

The invention relates to an automatic, non invasive blood pressure monitor for the non invasive measurement of the blood pressure according to the preamble of the claim 1.

Automatic electronic blood pressure monitors are known. These monitors determine the values of the systolic and the diastolic blood pressure e.g. on the basis of an oscillometric measurement. Other measurements are also known which are, may be based on electronic detection of Korotkoff sounds.

Automatic, electronic blood pressure measuring devices are usually applied on the upper arm of a patient, around the wrist or on a finger of the patient. While the measurement as such has proved to be sufficiently accurate with such devices, errors may be due to the fact that a user is making movements during the measurement. Ideally, the blood pressure should be taken when the user is sitting down and has been at rest for some time.

It is a disadvantage of known blood pressure measuring devices, that wrong measurement values may be given if the device is not properly used, especially if the user did not rest sufficiently before the measurement.

US 5,649,536 discloses a blood pressure monitor which is designed to make a single measurement of blood pressure values and to continuously determine blood pressure in a monitoring mode. Operation between these modes can be switched manually.

US 5,752,919 discloses a blood pressure monitor which is designed to run in a monitoring mode.

JP-A-7-227382 discloses an apparatus for continuously measuring blood pressure. Repeatedly measured blood pressure values and the dimension of the synchronous wave of heart pulses are graphically displayed along a common time axis. The document further discloses the possibility of performing one single blood pressure measurement. JP 04-227227 discloses a blood pressure monitor on which the preamble of claim 1 is based.

It is an object of the present invention to overcome the drawbacks of the prior art, especially to provide an automatic, non invasive blood pressure monitor which allows reduction of measurement errors which are based on the fact that a user is moving during the measurement or does not rest sufficiently before the measurement.

Still a further object of the invention is to provide a non invasive blood pressure monitor and a method for measuring the blood pressure which also allow fast determination of the blood pressure, if desired.

According to the present invention, these objects are solved with an automatic, non invasive blood pressure monitor having the features of the characterising portion of claim 1.

The blood pressure monitor according to the present invention is operatable in a first operating mode and in a second operating mode. In the first operating mode, the monitor is designed to determine and display blood pressure values on the basis of one single measurement in a conventional manner.

In the second operating mode, the blood pressure values are calculated on the basis of a series of measurements. The inventive device can be used in the first operating mode for a quick and rough blood pressure measurement. The second operating mode allows a much more precise measurement. During a certain period of time, measurements are repeated and the value of the systolic and the diastolic blood pressure is determined and disposed as an average of several measurements. The second operating mode is especially advantageous because the measurement series lasts for a certain amount of time. While the first measurement may be still erroneous because of insufficient rest of the user, the following measurements will be more accurate because the user will normally sit or lay down during the measurements.

The blood pressure monitor comprises a switch for switching between the first operating mode and the second operating mode. The user may at its discretion choose if a fast rough measurement or slower and more accurate measurement shall be made.

The blood pressure monitor comprises means for calculating or determining a statistical distribution of the measured values and means for forming an average of a number of measured blood pressure values.

The means for forming the average are programmed in such a way that a weighted average may be calculated.

It must be noted, that the first and the second operating mode are independent from each other. The blood pressure monitor could only be operated in the first operating mode or in the second operating mode if this should be desired by the user. "First" and "second" does not relate to time sequences or a specific order or preference of the operating modes and it does not exclude other operating modes.

In the second operating mode, a pause is preferably made between subsequent measurements. The pause can be about 60 seconds. This pause is advantageous in that it allows the measurement site (e.g. the upper arm or the wrist) to relax between measurements and in that it leads to an additional rest of the user.

Depending on the distribution, different calculation modes for calculating the average may be used. If one or more of the measured values substantially differs from the other measured values, a weighted average may be calculated. If all measured values are within a pre-determinable range, a normal, arithmetic average will be calculated.

In addition, if one or more of the measured values substantially differ from the others, an additional measurement may be made.

The blood pressure monitor according to the present invention is also adapted to calculate the pulse rate with high accuracy. As the measurement lasts for quite a long time, a precise mean value of the pulse rate pressure may be calculated.

The invention will be more clearly understood with reference to the drawings, which show:

Figure 1     a perspective representation of a blood pressure measuring device according to the invention,

Figure 2     a flow chart of the first operating mode,

Figure 3     a flow chart of the second operating mode and

Figure 4     a flow chart of the second operating mode of an alternative embodiment.

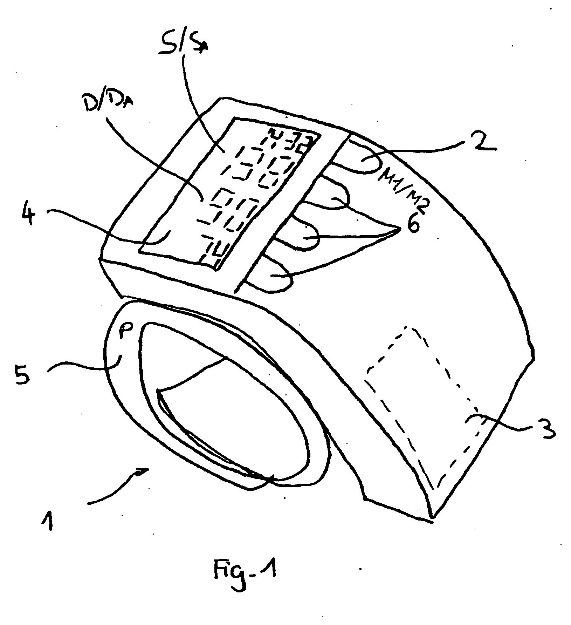

Figure 1 shows a blood pressure measuring monitor 1. The blood pressure measuring monitor 1 substantially consists of a housing 10 and a cuff 5. The housing 10 comprises a digital display 4 for displaying measurement results such as the pulse rate, the systolic blood pressure or the diastolic blood pressure. The blood pressure monitor 1 further comprises a calculating arrangement 3 for calculating the blood pressure values and the pulse rate based on the pressure P within the cuff 5. The pressure P may be measured in a conventional way with a pressure sensor (not shown).

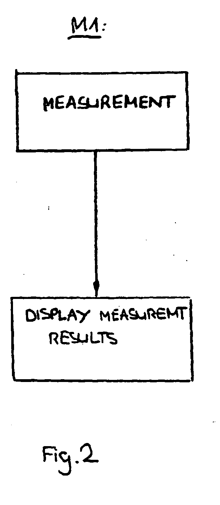

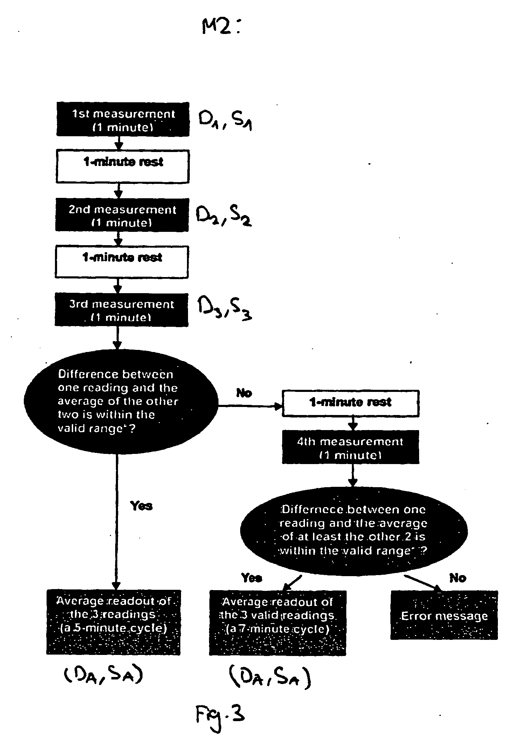

According to the invention, the blood pressure monitor is provided with a switch 2 for switching between a first operating mode M1 (see Figure 2) and a second operating mode M2 (see Figure 3) .

Additional buttons 6 are provided for resetting/turning on or off the device or starting the operation.

The blood pressure monitor 1 can be used either in the first operating mode M1 or in the second operating mode M2. In the first operating mode M1, the blood pressure measuring device is operating in a conventional manner. The value D of the diastolic blood pressure and the value S of the systolic blood pressure are measured in one single measurement cycle. This means, that the cuff is inflated once and the pressure P within the cuff is measured during one deflation cycle.

In the second operating mode, a number of measurements is made. In each of this measurements, a actual value of the diastolic blood pressure D and of the systolic blood pressure S is determined and stored within the blood pressure monitor 1. After the last of the measurements, an average value $D_A$ of the diastolic blood pressures and an average value $S_A$ of the systolic blood pressures is calculated in the calculating arrangement 3.

In Figure 2, a schematic representation of a measurement in the first operating mode M1 is shown. One single measurement is taken. The measurement result is immediately displayed on the display 4.

In Figure 3, a schematic representation of the second operating mode M2 is shown.

Three measurements are taken. Each measurement lasts for about 60 seconds. Between two subsequent measurement, a rest of another 60 seconds is made. This is necessary because between two measurements the venous re-flow has to be settled. This usually takes a minimum time of about 60 seconds. The time might be somewhat lower (50 seconds) or, of course, somewhat longer. The three measurements lead to three values D1, S1, D2, S2, D3, S3 of the systolic and the diastolic blood pressure, respectively.

After the third measurement, a comparison of the measured values is made. If all values are within a pre-determinable range, an average $D_A$, $S_A$ of the measured values is calculated.

The average of the diastolic blood pressure $D_A$ is formed as the arithmetic average of D1, D2 and D3. The average of the systolic blood pressure $S_A$ is formed as the arithmetic average S1, S2 and S3.

The valid range is about 20 mmHg for the systolic blood pressure and 12 mmHg for the diastolic blood pressure. This measurement cycle will take about 5 minutes.

If the measurement results of the three first measurements are not within the valid range, an additional measurement may be made. Usually, under physiological aspects, all measurement values shall be considered. Only in the case of errors due to motion artefacts or in case of an abnormal physiologic variation of the measurement results, one single result may be disregarded.

Usually, a variation of the measurement results during the three or four measurements does not necessarily lead to a rejection. People with unstable haemodynamic conditions often have systolic pressures which are outside the above mentioned pre-determinable range. Usually, these measurements are taken into consideration for calculating the average.

If the three first measurements are not within the valid range, in a first embodiment, an additional fourth measurement is made after a one minute rest. If the difference between one reading and the average of at least the other two is within the valid range, an arithmetic average of the three valid readings may be formed. This calculation leads to a seven minutes cycle. If no reading is within the valid range as compared to the average of the other two, an error message will be generated.

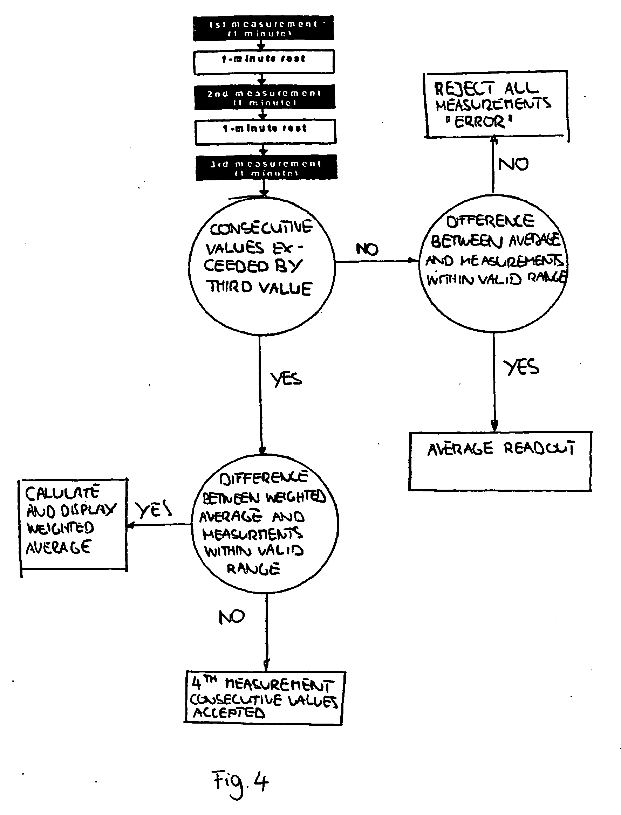

Usually, all artefact free results need to be accepted. If three artefact free results show a high variation (the difference between one reading and the average of the other two is outside the valid range), an additional average calculating mode may be applied (see Figure 4).

If two consecutive values are within the valid range and a third value (e.g. the first or the last one) exceeds the valid range, the third value is compared with a weighted average. If the difference between the third value and the weighted average is still outside the valid range, the first two values will be accepted and a fourth measurement will be made.

If there are no two successive valid measurements, the values will be rejected. This applies when an excessive result is between two valid measurements.

The following examples will show how different measurement results will be treated. The examples are given for systolic pressures. Similar calculations apply for the diastolic pressure.

Example 1:

S1 = 100, S2 = 100, S3 = 161

In this case, two consecutive valid values are exceeded by one abnormal value (S3). In this case, the weighted average applies.

$$A_W = \frac{((2 \cdot 100) + (2 \cdot 100) + 161)}{5} = 112$$

Since the difference between the third value and the weighted average is outside the valid range, a fourth measurement will be necessary in a similar way as shown in Figure 3.

Example 2:

S1 = 100, S2 = 161, S3 100

As there are no consecutive similar values, a non weighed average shall be formed (average = 120.33). The difference between all values and this average is outside the valid range. The whole measurement is not accepted because the patient's haemodynamic stability is not sufficient.

Example 3:

S1 = 161, S2 = 100, S3 = 100

This example is similar to example 1. The weighted average formula is applied. A fourth measurement is necessary.

Example 4:

S1 = 120, S2 = 150, S3 = 180

No consecutive values are found in this example. The normal average will be used. This patient does not have a haemodynamic stability necessary for a reasonable measurement. The average result will not be accepted and an error code will be displayed.

Example 5:

S1 = 120, S2 = 130, S4 = 140

All values are consecutive (which means there is no substantial difference between subsequent values). The normal non-weighted average is calculated. The average is 130. The difference between the average and all readings is within the valid range. An average value will be calculated and displayed.

The sequence of the measurement is schematically shown in Figure 4.

As mentioned above, errors due to artefacts are rejected. Technical artefacts may be detected on the basis of pulse analysis.

Physiologic rejections may be determined on the basis of statistic considerations.

The above algorithms show a possibility for a sophisticated calculation of an average value for the blood pressure values. This is basically possible with a blood pressure measuring device which is operatable in the first operating mode M1 (which allows fast and rough measurement) and in the second operating mode M2 (which allows a precise average measurement).

**Claims**

1. An automatic, non invasive blood pressure monitor (1) for indicating the diastolic and the systolic blood pressure ($D_A$), ($S_A$); (D), (S) of a patient, wherein
   the blood pressure monitor (1) is operable in a first operating mode (M1) and in a second operating mode (M2), and comprises means (2) for switching between the first operating mode (M1) and the second operating mode (M2),
   whereby the monitor is designed to determine and display the blood pressure values (D, S) on the basis of a single measurement when operating in the first operating mode (M1) and
   wherein the monitor is designed to calculate and display blood pressure values ($D_A$, $S_A$) as an average of a plurality of measured values of the diastolic and the systolic blood pressure (D1, D2, D3, S1, S2, S3) which are taken during a defined period of time when operating in the second operating mode (M2)
   **characterized in that** the means for switching are a switch and **in that** the monitor comprises means for calculating (3) a distribution of the values and means for forming a weighted average of the measured values (D1, D2, D3, S1, S2, S3).

2. A blood pressure monitor according to claim 1, **characterized in that** the monitor is designed to make a pause, preferably of about 60 seconds, between subsequent measurements, when operating in the second operating mode (M2).

3. A blood pressure monitor according to one of the claims 1 to 2, **characterized in that** it has means to form a difference between the measured values and the average of the values and **in that** it has means to compare the difference with a pre-determinable range.

4. A blood pressure monitor according to claim 3, **characterized in that** it has means to form a weighted average for comparison if one of the measured values substantially differs from the others.

5. A blood pressure monitor according to claim 4, **characterized in that** it is designed to make an additional measurement if the difference between the substantially differing measurement and the weighted average is outside a pre-determinable range and **in that** it is designed to display a weighted average if the difference is within the pre-determinable range.

6. A blood pressure monitor according to one of the claims 1 to 5, **characterized in that** it comprises means to calculate a pulse rate.

**Patentansprüche**

1. Automatisches, nichtinvasives Blutdruckmessgerät (1) zur Angabe des diastolischen und systolischen Blutdrucks ($D_A$), ($S_A$); (D), (S) eines Patienten, wobei
   das Blutdruckmessgerät (1) in einem ersten Betriebsmodus (M1) und einem zweiten Betriebsmodus (M2) betrieben werden kann und Mittel (2) zum Umschalten zwischen dem ersten Betriebsmodus (M1) und dem zweiten Betriebsmodus (M2) aufweist,
   wobei das Messgerät zur Bestimmung und Anzeige der Blutdruckwerte (D, S) auf Grundlage einer Einzelmessung bei Betrieb im ersten Betriebsmodus (M1) ausgelegt ist, und
   wobei das Gerät zur Berechnung und Anzeige von Blutdruckwerten ($D_A$, $S_A$) als Mittelwert mehrerer, bei Betrieb im zweiten Betriebsmodus (M2) über einen festgelegten Zeitraum hinweg erhobener Messwerte des diastolischen und systolischen Blutdrucks (D1, D2, D3, S1, S2, S3) ausgelegt ist,
   **dadurch gekennzeichnet, dass**
   das Mittel zum Umschalten ein Schalter ist, und dass das Messgerät Mittel zur Berechnung (3) einer Werteverteilung und Mittel zur Bildung eines gewichteten Mittels der Messwerte (D1, D2, D3, S1, S2, S3) aufweist.

2. Blutdruckmessgerät nach Anspruch 1, **dadurch gekennzeichnet, dass** das Messgerät bei Betrieb im zweiten Betriebsmodus (M2) für das Einhalten eines zeitlichen Abstands, vorzugsweise von etwa 60 Sekunden, zwischen Folgemessungen ausgelegt ist.

**3.** Blutdruckmessgerät nach einem der Ansprüche 1 bis 2, **dadurch gekennzeichnet, dass** es Mittel zur Bildung einer Differenz zwischen den Messwerten und dem Mittelwert der Werte sowie Mittel zum Vergleichen der Differenz mit einem zuvor festlegbaren Bereich aufweist.

**4.** Blutdruckmessgerät nach Anspruch 3, **dadurch gekennzeichnet, dass** es Mittel zur Bildung eines gewichteten Mittels zwecks Vergleichs, ob einer der Messwerte wesentlich von den anderen abweicht, aufweist.

**5.** Blutdruckmessgerät nach Anspruch 4, **dadurch gekennzeichnet, dass** es bei Vorliegen einer außerhalb eines zuvor festlegbaren Bereichs liegenden Differenz zwischen der wesentlich abweichenden Messung und dem gewichteten Mittel zur Durchführung einer zusätzlichen Messung und bei Vorliegen einer innerhalb des zuvor festlegbaren Bereichs liegenden Differenz zur Anzeige eines gewichteten Mittels ausgelegt ist.

**6.** Blutdruckmessgerät nach einem der Ansprüche 1 bis 5, **dadurch gekennzeichnet, dass** es Mittel zur Berechnung einer Pulsfrequenz aufweist.

## Revendications

**1.** Appareil automatique non invasif de surveillance de la pression sanguine (1) destiné à indiquer la pression sanguine diastolique et systolique ($D_A$), (SA) ; (D), (S) d'un patient, dans lequel
l'appareil de surveillance de la pression sanguine (1) est actionnable dans un premier mode de fonctionnement (M1) et dans un second mode de fonctionnement (M2), et comprend des moyens (2) destinés à commuter entre le premier mode de fonctionnement (M1) et le second mode de fonctionnement (M2),
moyennant quoi l'appareil de surveillance est conçu pour déterminer et afficher les valeurs de la pression sanguine (D, S) sur la base d'une mesure unique lorsque l'appareil fonctionne dans le premier mode de fonctionnement (M1), et moyennant quoi l'appareil de surveillance est conçu pour calculer et afficher les valeurs de la pression sanguine ($D_A$, $S_A$) en tant que moyenne d'une pluralité de valeurs mesurées de la pression sanguine diastolique et systolique (D1, D2, D3, S1, S2, S3) qui sont prises pendant une période de temps définie lorsque l'appareil fonctionne dans le second mode de fonctionnement (M2),
**caractérisé en ce que** des moyens (2) destinés à commuter sont des commutateurs,
et **en ce que** l'appareil de surveillance comprend des moyens destinés à calculer (3) une répartition des valeurs et des moyens destinés à former une moyenne pondérée des valeurs mesurées (D1, D2, D3, S1, S2, S3).

**2.** Appareil de surveillance de la pression sanguine selon la revendication 1, **caractérisé en ce que** l'appareil de surveillance est conçu pour effectuer une pause, de préférence d'environ 60 secondes, entre les mesures ultérieures lorsqu'il fonctionne dans le second mode de fonctionnement (M2).

**3.** Appareil de surveillance de la pression sanguine selon l'une quelconque des revendications 1 à 2, **caractérisé en ce qu'**il possède des moyens pour former une différence entre les valeurs mesurées et la moyenne des valeurs et **en ce qu'**il présente des moyens pour comparer la différence avec une gamme pouvant être prédéterminée.

**4.** Appareil de surveillance de la pression sanguine selon la revendication 3, **caractérisé en ce qu'**il présente des moyens pour former une moyenne pondérée pour une comparaison si une des valeurs mesurées diffère sensiblement des autres.

**5.** Appareil de surveillance de la pression sanguine selon la revendication 4, **caractérisé en ce qu'**il est conçu pour effectuer une mesure supplémentaire si la différence entre la mesure sensiblement différente et la moyenne pondérée est en dehors d'une gamme pouvant être prédéterminée et **en ce qu'**il est conçu pour afficher une moyenne pondérée si la différence est à l'intérieur de la gamme pouvant être prédéterminée.

**6.** Appareil de surveillance de la pression sanguine selon l'une quelconque des revendications 1 à 5, **caractérisé en ce qu'**il comprend des moyens pour calculer un rythme cardiaque.

Fig-1

M1:

```
┌─────────────────────────┐
│                         │
│     MEASUREMENT         │
│                         │
└─────────────────────────┘
            │
            │
            ▼
┌─────────────────────────┐
│  DISPLAY MEASUREMT      │
│     RESULTS            │
│                         │
└─────────────────────────┘
```

Fig. 2

M2 :

```
┌─────────────────────┐
│   1st measurement   │   D₁, S₁
│     (1 minute)      │
└─────────────────────┘
          ↓
┌─────────────────────┐
│    1-minute rest    │
└─────────────────────┘
          ↓
┌─────────────────────┐
│   2nd measurement   │   D₂, S₂
│     (1 minute)      │
└─────────────────────┘
          ↓
┌─────────────────────┐
│    1-minute rest    │
└─────────────────────┘
          ↓
┌─────────────────────┐
│   3rd measurement   │   D₃, S₃
│     (1 minute)      │
└─────────────────────┘
          ↓
    Difference between              ┌─────────────────────┐
    one reading and the    No       │    1-minute rest    │
    average of the other  ──────→   └─────────────────────┘
    two is within the                         ↓
    valid range ?                   ┌─────────────────────┐
          │                         │   4th measurement   │
          │ Yes                     │     (1 minute)      │
          │                         └─────────────────────┘
          │                                   ↓
          │                          Differnece between one
          │                          reading and the average
          │                          of at least the other 2 is
          │                          within the valid range ?
          │                           Yes              No
          ↓                            ↓                ↓
┌─────────────────────┐  ┌─────────────────────┐  ┌─────────────────────┐
│ Average readout of  │  │ Average readout of  │  │                     │
│  the 3 readings     │  │ the 3 valid readings│  │   Error message     │
│  (a 5-minute cycle) │  │  (a 7-minute cycle) │  │                     │
└─────────────────────┘  └─────────────────────┘  └─────────────────────┘
     (D_A, S_A)               (D_A, S_A)
```

$(D_A, S_A)$   $(D_A, S_A)$

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5649536 A
- US 5752919 A
- JP 7227382 A
- JP 4227227 A